# EUROPEAN PATENT SPECIFICATION

(11) **EP 2 543 342 B1**
(45) Date of publication and mention of the grant of the patent: **13.11.2019**
(21) Application number: 11750519.8
(22) Date of filing: 23.02.2011
(51) Int. Cl.: A61M 1/36, A61F 2/06

(54) **ARTIFICIAL BLOOD VESSEL**
KÜNSTLICHES BLUTGEFÄSS
VAISSEAU SANGUIN ARTIFICIEL

(30) Priority: 04.03.2010 JP 2010047282
(43) Date of publication of application: 09.01.2013
(73) Proprietor: Terumo Kabushiki Kaisha, Tokyo 151-0072 (JP)
(72) Inventor: TANI Kazuyoshi, Fujinomiya-shi Shizuoka 418-0015 (JP); SAWADA Akira, Fujinomiya-shi Shizuoka 418-0015 (JP); TAKAHASHI Yoshikazu, Fujinomiya-shi Shizuoka 418-0015 (JP)
(74) Representative: Dossmann, Gérard
(86) International application number: PCT/JP2011/053916
(87) International publication number: WO 2011/108409

(56) References cited:
- EP-A2- 0 604 803
- WO-A1-2006/013234
- WO-A1-2009/082718
- JP-A- 2005 511 200
- US-A- 4 501 263
- US-A- 6 090 067
- US-A1- 2003 033 005
- US-A1- 2003 065 343
- US-A1- 2005 171 479
- US-A1- 2007 067 014
- US-A1- 2007 250 154

## Description

### Technical Field

The present invention relates to an artificial blood vessel to be used to cure an aorta or the like.

### Background Art

A common way of curing aortic diseases, such as aortic aneurysm and aortic dissection, is by surgery involving replacement of the aortic arch with an artificial blood vessel. There have been developed various artificial blood vessels for this purpose (as disclosed in U.S. Patent No. 6770090, for example).

The surgery mentioned above needs a heart-lung machine for extracorporeal circulation. Moreover, the one that ranges from the ascending aorta or aortic arch to the descending aorta is greatly invasive, imposing a large burden on the patient, because of its extended incision Blood vessel grafts with inserting ports are known from US6090067.

### Summary of Invention

There have been contrived various techniques that will supersede the above-mentioned surgery involving replacement. One of them is intended to insert a stent graft into the aorta by partially clamping the ascending aorta, connecting an artificial blood vessel that bypasses the right branchiocephalic arterial trunk or left common carotid artery branching from the aortic arch, and finally closing the branching part of the aortic arch.

For reduction of burden and invasion on the patient, the foregoing technique should permit easy and smooth insertion of intravascular curing devices (e.g., a delivery system for placement of a stent graft and a contrast catheter to ensure the state of placement of the stent graft) into the aorta.

The present invention as defined in claim 1 was completed to address the problems involved in the foregoing conventional technique. Thus, it is an object of the present invention to provide an artificial blood vessel that permits easy and smooth insertion of intravascular curing devices, with more reduced burden and invasion on the patient.

The artificial blood vessel according to the present invention includes one proximal-end and at least one distal-end communicating with the proximal-end and an inserting port positioned between them, the inserting port being provided with a check valve that permits an intravascular curing device to pass through up to the proximal-end and also prevents body fluid from flowing out from the artificial blood vessel.

As mentioned above, the artificial blood vessel according to the present invention is composed of one proximal-end and at least one distal-end communicating with the proximal-end and an inserting port positioned between them, the inserting port being provided with a check valve. The advantage of this structure is that the blood vessel branching from the aortic arch can be bypassed while the heart is beating, without the necessity of extracorporeal circulation through a heart-lung machine, by connecting the proximal-end to the ascending aorta, which is partially clamped, and connecting the distal-end to the blood vessel branching from the aortic arch. This leads to a reduced burden on the patient. Another advantage is that the inserting port permits smooth insertion and advance of a stent graft into the aortic arch. In this way it is possible to insert an intravascular curing device, such as delivery catheter, more easily and smoothly than inserting it from the groin of the thigh.

One way of inserting a stent graft into the aortic arch, with the artificial blood vessel attached to the aorta and the blood vessel branching from the aortic arch closed, is accomplished by suturing the proximal-end of the artificial blood vessel to the ascending aorta, which is partially clamped while the heart is beating, dissecting the blood vessel, such as right branchiocephalic arterial trunk, branching from the aortic arch and suturing the distal-end of the artificial blood vessel to the dissected blood vessel, thereby closing the dissected blood vessel branching from the aortic arch, inserting a delivery catheter from the inserting port such that the delivery catheter passes through the proximal-end and advances to the aortic arch, which is the object position, thereby inserting the stent graft into the aortic arch, and removing the delivery catheter from the inserting port, followed by cutting off the inserting port and closing the cut part.

The distal-end of the artificial blood vessel may have a branched structure or three-forked structure, so that it may be sutured to more than one blood vessel. This makes the artificial blood vessel more compatible with the living body.

The inserting port mentioned above should be positioned between the proximal-end and the branching distal-end. This structure facilitates insertion of an intravascular curing device into the aorta or other blood vessels through the proximal-end.

The check valve mentioned above is composed of first and second valve components, the first valve component preventing body fluid from flowing out while the intravascular curing device is inserted into the inserting port, and the second valve component preventing body fluid from flowing out while the intravascular curing device is not yet inserted into the inserting port. The check valve in this structure ensures good sealing performance regardless of whether or not the intravascular curing device is inserted into the inserting port.

The check valve composed of first and second valve should be constructed as follows. The second valve component is positioned closer to the front side for insertion of the intravascular curing device than the first valve component. The first valve component has a hole permitting the intravascular curing device to be smoothly inserted and to come into close contact with the external surface of the intravascular curing device which has been inserted. The second valve component has a pair of walls inclined toward the direction in which the intravascular curing device is inserted into the inserting port. Adjacent ends of the inclined walls come close each other and separate from each other so that the intravascular curing device passes through the space between the inclined walls. The thus constructed check valve is simple in structure but is capable of tight sealing regardless of whether or not the intravascular curing device is inserted into the inserting port.

The artificial blood vessel may be constructed such that the proximal-end is capable of connection to the ascending aorta and the distal-end is capable of connection to at least one of the right branchiocephalic arterial trunk, left common carotid artery, and left subclavian artery.

According to the present invention, the artificial blood vessel is composed of one proximal-end and at least one distal-end communicating with the proximal-end and an inserting port positioned between them, the inserting port being provided with a check valve. The advantage of this structure is that the blood vessel branching from the aortic arch can be bypassed while the heart is beating, without the necessity of extracorporeal circulation through a heart-lung machine, by connecting the proximal-end to the ascending aorta, which is partially clamped, and connecting the distal-end to the blood vessel branching from the aortic arch. This leads to a reduced burden on the patient. Another advantage is that the inserting port permits smooth insertion and advance of a stent graft into the aortic arch. In this way it is possible to insert an intravascular curing device, such as delivery catheter, more easily and smoothly than inserting it from the groin of the thigh. Brief Description of Drawings

[Fig. 1]

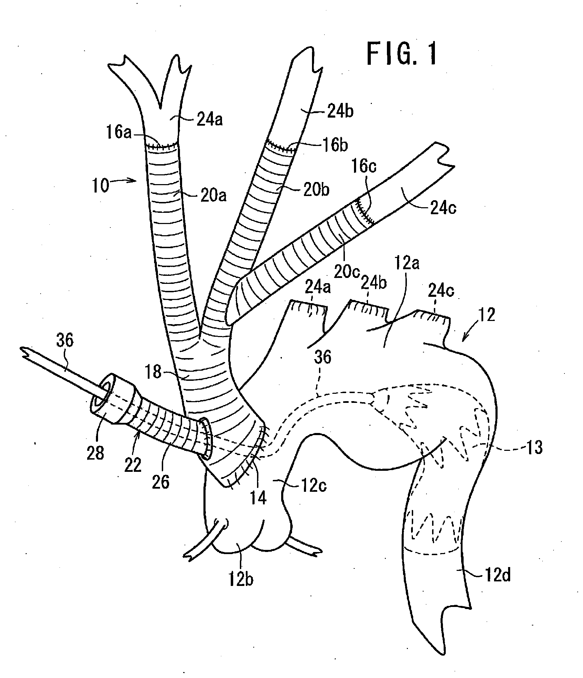

   Fig. 1 is a schematic diagram illustrating the artificial blood vessel pertaining to one embodiment of the present invention which is applied to the living body.
[Fig. 2]

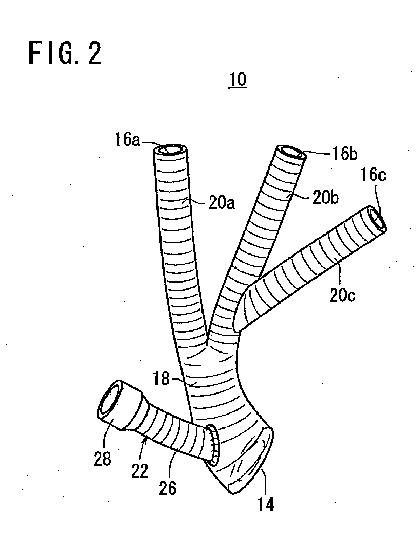

   Fig. 2 is a perspective view showing the entire structure of the artificial blood vessel.
[Fig. 3]

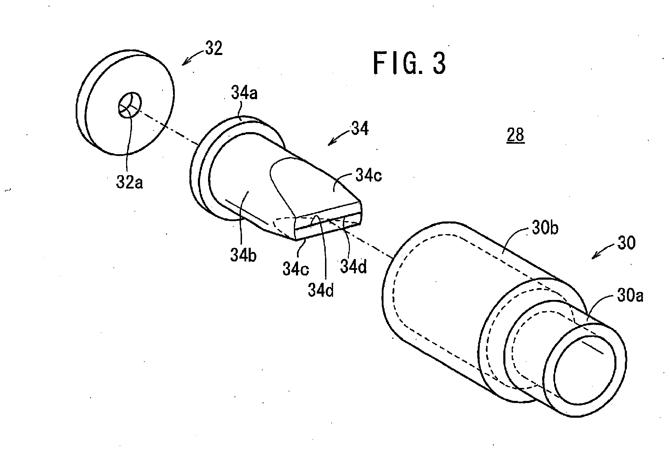

   Fig. 3 is an exploded perspective view showing the structure of the check valve attached to the inserting port.
[Fig. 4]

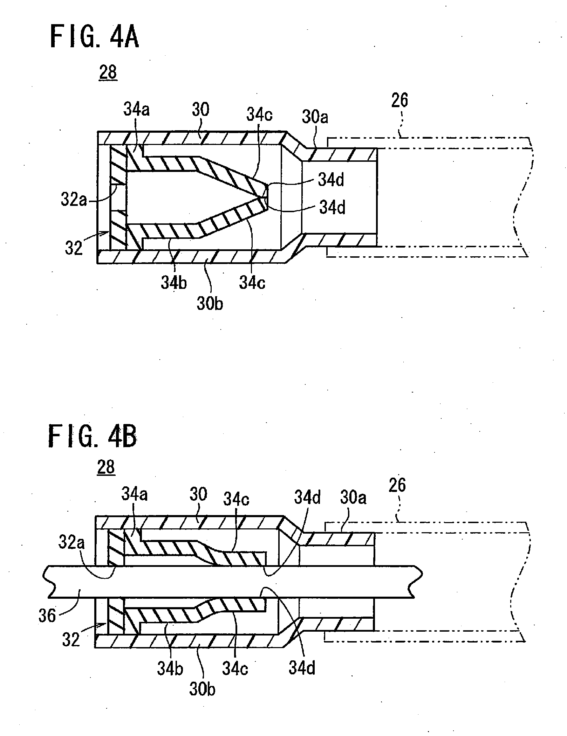

   Fig. 4A is a side sectional view (in the axial direction) of the check valve, with the intravascular curing device not inserted and Fig. 4B is a side sectional view, in the axial direction, of the check valve, with the intravascular curing device inserted.
[Fig. 5]

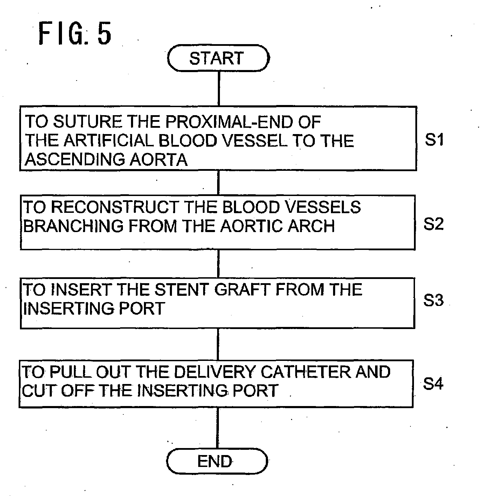

   Fig. 5 is a flow chart showing the procedure for attaching the artificial blood vessel to the aorta and then placing a stent graft at a desired position in the aorta by using the artificial blood vessel.
[Fig. 6]

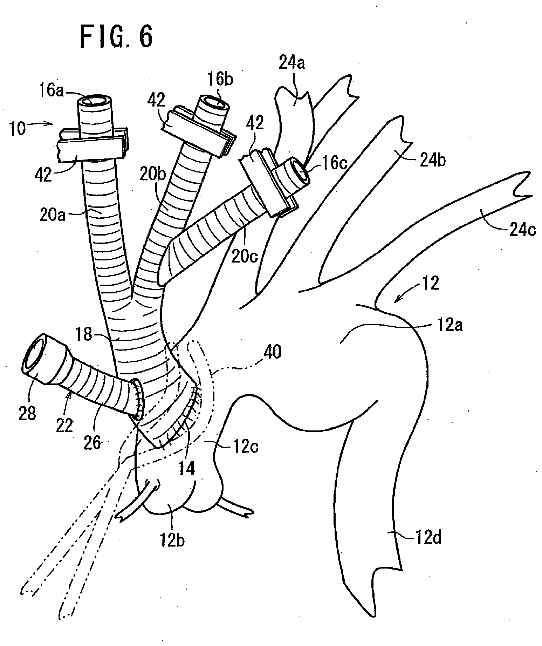

   Fig. 6 is a diagram illustrating the artificial blood vessel, with its proximal-end connected to the ascending aorta.
[Fig. 7]

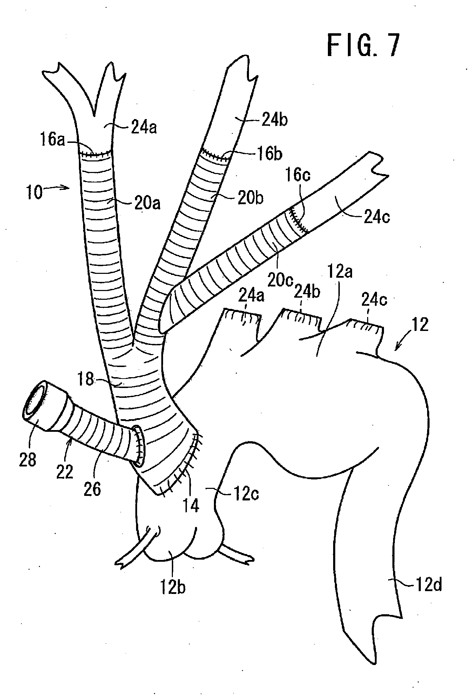

   Fig. 7 is a diagram illustrating the reconstruction of the blood vessels branching from the aortic arch.
[Fig. 8]

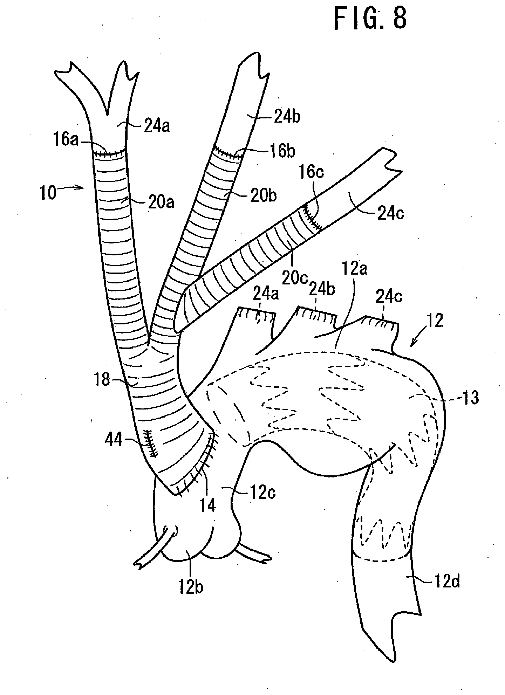

   Fig. 8 is a diagram illustrating the artificial blood, with the inserting port cut off, which remains attached to the aorta after insertion of a stent graft. Mode for Carrying Out the Invention

The following is a detailed description (referring to the accompanying drawings) of the preferred embodiment of the artificial blood vessel pertaining to the present invention.

Fig. 1 is a schematic diagram illustrating the artificial blood vessel 10 pertaining to one embodiment of the present invention which is applied to the living body. Fig. 2 is a perspective view showing the entire structure of the artificial blood vessel 10. The artificial blood vessel 10 pertaining to the embodiment of the present invention is used to cure the blood vessel of the living body, such as the aortic arch 12a of the aorta 12, by insertion and placement of the stent graft 13 in the aortic arch. The artificial blood vessel 10 may also be used to cure other parts of the living body, as a matter of course.

As shown in Figs. 1 and 2, the artificial blood vessel 10 is composed of one proximal-end 14 in large diameter and three distal-ends 16a, 16b, and 16c communicating with the proximal-end 14, the trunk tube 18 connecting the proximal-end 14 to the distal-end 16a and 16b, the branch tubes 20a, 20b, and 20c branching off from the trunk tube 18, and the inserting port 22 attached to the trunk tube 18.

It is to be noted from Fig. 1 that the artificial blood vessel 10 is shaped with specific dimensions so that the proximal-end 14 can be connected to the ascending aorta 12c between the aortic arch 12a and the aortic valve 12b and the distal-end 16a, 16b, and 16c can be connected respectively to the right branchiocephalic arterial trunk 24a, the left common carotid artery 24b, and the left subclavian artery 24c which branch off from the aortic arch 12a. To be more specific, the trunk tube 18 has an outside diameter of about 12 mm to 30 mm, a wall thickness of about 0.1 mm to 1 mm, and a length of about 10 mm to 100 mm. Each of the branch tubes 20a, 20b, and 20c has an outside diameter of about 6 mm to 10 mm, a wall thickness of about 0.1 mm to 1 mm, and a length of about 10 mm to 150 mm. The trunk tube 18 and the branch tubes 20a, 20b, and 20c may be formed from the same material as used for known artificial blood vessels. Such material includes, for example, polyester fiber and ePTFE (expanded polytetrafluoroethylene).

The inserting port 22 is composed of the port tube 26 and the check valve 28. The port tube 26 is connected to that part (or the trunk tube 18 in this case) between the proximal-end 14 and each of the distal-end 16a and 16b. The check valve 28 is attached to that part of the port tube 26 which is opposite to that part of the port tube 26 which is connected to the trunk tube 18. The port tube 26 may be formed from the same material as used for the trunk tube 18 and the branch tubes 20a and 20b. It may have an outside diameter of about 6 mm to 16 mm, a wall thickness of about 0.1 mm to 1 mm, and a length of about 10 mm to 150 mm.

The check valve (or hemostatic valve) 28, shown in Figs. 3, 4A, and 4B, is composed of the housing 30 and the first and second valve components 32 and 34. The housing 30 is a stepped cylindrical body made of plastics material, with a smaller diameter portion 30a fitting into the opening of the port tube 26. The first and second valve components 32 and 34 are arranged in the axial direction in a larger diameter portion 30b of the housing 30.

The first valve component 32 is a discoid member having a hole 32a at its center. It is made of an elastic material such as rubber, so that it fluid-tightly fits to the inner wall of the housing 30. The hole 32a has an inner diameter large enough for the delivery catheter 36 (as an intravascular curing device) to pass through and tightly hold the delivery catheter 36 passing through it. The delivery catheter 36 is intended to deliver the balloon catheter or the stent graft 13. (See Figs. 1 and 4B.)

The first valve component 32 is an ordinary rubber stopper and hence the hole 32a remains open while the delivery catheter 36 does not pass through it. Therefore, it cannot prevent body fluid (blood) from flowing out from the port tube 26 (or the aorta 12).

The second valve component 34 is arranged in the housing 30 next to the first valve component 32 in the direction in which the delivery catheter 36 is inserted. The second valve component 34 consists of a discoid section 34a, a cylindrical section 34b, and a pair of inclined walls 34c and 34c. The discoid section 34a is fluid-tightly fixed to the inner wall of the housing 30. The cylindrical section 34b joins together the discoid section 34a and the inclined walls 34c and 34c. The inclined walls 34c and 34c are inclined toward the direction in which the delivery catheter 36 is inserted. These sections are formed from an elastic material such as rubber. Incidentally, the cylindrical section 34b may be omitted.

As shown in Fig. 3, the paired inclined walls 34c and 34c are formed such that their adjacent ends 34d and 34d are straight and in close contact with each other. Therefore, the paired inclined walls 34c and 34c keep the adjacent ends 34d and 34d in close contact with each other while the delivery catheter 36 is not inserted into the check valve 28, as shown in Figs. 3 and 4A. In this state, the inclined walls 34c and 34c receive the pressure of body fluid (blood) through the port tube 26. This pressure tightly closes the adjacent ends 34d and 34d. On the other hand, when the delivery catheter 36 is inserted into the check valve 28, the adjacent ends 34d and 34d are separated from each other by the delivery catheter 36 that passes between them, as shown in Fig. 4B. In this way the delivery catheter 36 can be inserted.

The second valve component 34 is of the type of so-called duckbill valve. Therefore, it functions as a check valve that prevents body fluid from flowing out from the port tube 26 while the delivery catheter 36 is not inserted, as shown in Fig. 4A. However, the straight adjacent ends 34d and 34d open when the delivery catheter 36 is inserted, so that the second valve component 34 hardly function as a check valve.

As mentioned above, the check valve 28 has the first valve component 32 at its proximal-end from which the delivery catheter 36 is inserted and the second valve component 34 in front of the first valve component 32. The first valve component 32 tightly seals while the delivery catheter 36 is inserted into the inserting port 22 (or the check valve 28), and the second valve component 34 tightly seals while the delivery catheter 36 is not inserted. This structure effectively prevents body fluid from flowing out regardless of whether or not the delivery catheter 36 is inserted.

The following is a description of the technique and function in attaching the above-mentioned artificial blood vessel 10 to the aorta 12.

Fig. 5 is a flowchart showing the procedure for attaching the artificial blood vessel 10 to the aorta 12 and for placing the stent graft 13 at a desired position in the aorta 12 by using the artificial blood vessel 10. The artificial blood vessel 10 is not intended to replace a portion of the aorta 12 but it is used to bypass the right branchiocephalic arterial trunks 24a etc. (branching from the aortic arch), with the ascending aorta 12c partially clamped.

Step S1 in Fig. 5 is to suture the proximal-end 14 of the artificial blood vessel 10 to the ascending aorta 12c (See Fig. 6). This suture is accomplished while partially clamping a portion of the ascending aorta 12c with the forceps 40 (while allowing the heart to beat) without extracorporeal circulation through a heart-lung machine. During Step 1, the distal-end tubes 20a, 20b, and 20c are kept closed by the forceps 42. Incidentally, the inserting port 22 is kept closed by the check valve 28.

Step S2 is intended to reconstruct the blood vessels branching from the aortic arch 12a, as shown in Fig. 7. That is, in this step, the right branchiocephalic arterial trunk 24a, the left common carotid artery 24b, and the left subclavian artery 24c are cut off from the aortic arch 12a, and they are sutured respectively to the distal-end 16a, 16b, and 16c of the branched tubes 20a, 20b, and 20c of the artificial blood vessel 10. The dissected parts are sutured and closed.

Step S3 is intended to insert the delivery catheter 36 from the inserting port 22 and advance the delivery catheter 36 through the port tube 26, the proximal-end tube 18, and the proximal-end 14 until it reaches the aortic arch 12a (the desired position), and release the stent graft 13 there, as shown in Fig. 1. In this way, the stent graft 13 is inserted and placed at a desired position between the aortic arch 12a and the descending aorta 12d.

Step S4 is intended to pull out the delivery catheter 36 from the inserting port 22 and then cut off the inserting port 22 from the proximal-end tube 18 and suture and close the cut part 44, as shown in Fig. 8. In this way, the artificial blood vessel 10 functions as the bypass for the right branchiocephalic arterial trunk 24a, the left common carotid artery 24b, and the left subclavian artery 24c which branch off from the aortic arch 12a, and the stent graft 13 functions as a prosthesis for the aortic arch 12a.

As mentioned above, the artificial blood vessel 10 according to the present invention is composed of one proximal-end 14 and three distal-end 16a, 16b, and 16c, each communicating with the proximal-end 14, and the inserting port 22 positioned between the proximal-end 14 and the distal-end 16a, 16b, and 16c, the inserting port 22 being provided with the check valve 28.

Therefore, the artificial blood vessel 10 permits the blood vessels branching from the aortic arch to be bypassed while the heart is beating without extracorporeal circulation through a heart-lung machine as the result of connecting the proximal-end 14 to the ascending aorta 12c, which is partially clamped, and the blood vessels branching from the aortic arch are connected to the distal-end 16a, 16b, and 16c. This procedure can be accomplished with less burden on the patient. Moreover, the artificial blood vessel 10 permits the stent graft 13 to be inserted and advanced to the aortic arch 12 through the inserting port 22. Thus it helps easy and smooth insertion of the intravascular curing device, such as the delivery catheter 36 (or delivery system) to place the stent graft 13 and the contrast catheter (not shown) to ensure the placement of the stent graft 13. This leads to a further reduction of burden on the patient.

The artificial blood vessel 10 has at least three-forked distal-end 16a, 16b, and 16c, which facilitates connection with the branching blood vessels. In other words, distal-end is branched into at least three sections, so that the distal-end is easily connected to a plurality of branching blood vessels. Because of this structure, the artificial blood vessel 10 is highly compatible with the living body.

The artificial blood vessel 10 has the inserting port 22 between the distal-ends (or the distal-end 16a, 16b, and 16c) and the proximal-end 14. The inserting port 22 facilitates insertion of the intravascular curing device into the blood vessel (e.g., aorta) through the proximal-end 14. Moreover, the inserting port 22 is positioned closer to the proximal-end 14 than the branched distal-end, so that it facilitates insertion of the intravascular curing device into the aorta 12. Incidentally, the inserting port 22 may be positioned at the distal-end tube 20a etc. depending on the technique employed, as a matter of course.

In addition, the inserting port 22 has the check valve 28 which is composed of the first valve component 32 and the second valve component 34, as mentioned above. The inserting port 22 of this structure easily achieves high sealing performance regardless of whether or not the delivery catheter 36 is inserted into the inserting port 22, thereby preventing body fluid from flowing out from the inserting port 22.

The present invention is not restricted to the embodiments mentioned above but may be variously modified and changed in its structure and procedure within the scope thereof, as a matter of course.

For example, the number of the distal-end 16a to 16c may be at least one or more according to the technique employed, and the number of the distal-end tubes 20a and 20b may be varied accordingly.

The check valve 28 attached to the inserting port 22 is not restricted in structure to the one which consists of the particular first valve component 32 and the second valve component 34 mentioned above. It may be of any structure as defined in claim 1 so long as it achieves high sealing performance regardless of whether or not the intravascular curing device is inserted into the inserting port 22.

## Claims

1. An artificial blood vessel (10) which comprises one proximal-end (14) and at least one distal-end (16a) communicating with said proximal-end (14),
wherein the artificial blood vessel (10) further comprises an inserting port (22) positioned between said proximal-end (14) and said distal-end (16a), sai
d inserting port (22) being provided with a check valve (28), wherein said check valve (28) is configured to permit an intravascular curing device (36) to pass through up to said proximal-end (14) and also prevent body fluid from flowing out from said artificial blood vessel (10), in which said check valve (28) has a first valve component (32) that prevents body fluid from flowing out when the intravascular curing device (36) is inserted through the inserting port (22) and a second valve component (34) that prevents body fluid from flowing out when the intravascular curing device (36) is not inserted through the inserting port (22).

2. The artificial blood vessel (10) according to Claim 1, in which the distal-end (16a) branches off.

3. The artificial blood vessel (10) according to Claim 2, in which said branching forms at least three-forked branches.

4. The artificial blood vessel (10) according to Claim 2 or 3, in which said inserting port (22) is positioned between said branch and said proximal-end (14).

5. The artificial blood vessel (10) according to Claim 1, in which
the second valve component (34) is positioned closer to the front side for insertion of the intravascular curing device (36) than the first valve component (32),
the first valve component (32) has a hole (32a) which permits the intravascular curing device (36) to be slidably inserted and comes into close contact with the external surface of the intravascular curing device (36) which has been inserted, and
the second valve component (34) has a pair of walls (34c) inclined toward the direction in which the intravascular curing device (36) is inserted into the inserting port (22), adjacent ends (34d) of said inclined walls (34c) are configured to come close each other and separate from each other so that the intravascular curing device (36) is capable to pass through the space between the inclined walls (34c).

6. The artificial blood vessel (10) according to Claim 1, in which
the proximal-end (14) is capable of connection to the ascending aorta (12c) and
the distal-end (16a) is capable of connection to at least one of the right branchiocephalic arterial trunk (24a), the left common carotid artery (24b), and the left subclavian artery (24c).

## Patentansprüche

1. Künstliches Blutgefäß (10), umfassend
ein proximales Ende (14) und mindestens ein distales Ende (16a), das mit dem proximalen Ende (14) verbunden ist,
wobei das künstliche Blutgefäß (10) ferner eine Einführungsöffnung (22) aufweist, die zwischen dem proximalen Ende (14) und dem distalen Ende (16a) angeordnet ist;
die Einführungsöffnung (22) ist mit einem Rückschlagventil (28) versehen,
wobei das Rückschlagventil (28) so konfiguriert ist, dass es ermöglicht wird, von einer intravaskulären Behandlungsvorrichtung (36) bis hin zum proximalen Ende (14) hindurchzutreten und außerdem das Austreten von Körperflüssigkeit aus dem künstlichen Blutgefäß (10) verhindert wird,
wobei das Rückschlagventil (28) ein erstes Ventilbauteil (32) aufweist, das das Austreten von Körperflüssigkeit verhindert, wenn die intravaskuläre Behandlungsvorrichtung (36) durch die Einführungsöffnung (22) eingeführt ist, und ein zweites Ventilbauteil (34), das das Austreten von Körperflüssigkeit verhindert, wenn die intravaskuläre Behandlungsvorrichtung (36) nicht durch die Einführungsöffnung (22) eingeführt ist.

2. Künstliches Blutgefäß (10) nach Anspruch 1, wobei das distale Ende (16a) abzweigt.

3. Künstliches Blutgefäß (10) nach Anspruch 2, wobei die Abzweigung mindestens drei Gefäßäste bildet.

4. Künstliches Blutgefäß (10) nach Anspruch 2 oder 3, wobei die Einführungsöffnung (22) zwischen den Gefäßästen und dem proximalen Ende (14) angeordnet ist.

5. Künstliches Blutgefäß (10) nach Anspruch 1, wobei
das zweite Ventilbauteil (34) näher an der Frontseite zum Einführen der intravaskulären Behandlungsvorrichtung (36) positioniert ist als das erste Ventilbauteil (32),
das erste Ventilbauteil (32) ein Loch (32a) aufweist, das ein verschiebbares Einsetzen der intravaskulären Behandlungsvorrichtung (36) ermöglicht und in enger Berührung mit der Außenfläche der eingesetzten intravaskulären Behandlungsvorrichtung (36) kommt, und
das zweite Ventilbauteil (34) ein Paar Wände (34c) aufweist, das in die Richtung geneigt ist, in welche die intravaskuläre Behandlungsvorrichtung (36) in die Einführungsöffnung (22) eingeführt wird,
wobei zueinander benachbarte Enden (34d) jener geneigten Wände (34c) so konfiguriert sind, dass sie sich einander nahe kommen und wieder voneinander getrennt werden können, so dass die intravaskuläre Behandlungsvorrichtung (36) durch den Raum zwischen den geneigten Wänden (34c) bewegt werden kann.

6. Künstliches Blutgefäß (10) nach Anspruch 1, wobei
das proximale Ende (14) mit der aufsteigenden Aorta (12c) verbindbar ist und
das distale Ende (16a), sich mit mindestens einem der folgenden Elemente verbinden kann: dem rechten Truncus brachiocephalicus (24a), und/oder der linken Halsschlagader (24b) und/oder der linken Schlüsselbeinarterie (24c).

## Revendications

1. Vaisseau sanguin artificiel (10) qui comprend une extrémité proximale (14) et au moins une extrémité distale (16a) communiquant avec ladite extrémité proximale (14),
dans lequel le vaisseau sanguin artificiel (10) comprend en outre un orifice d'insertion (22) positionné entre ladite extrémité proximale (14) et ladite extrémité distale (16a),
ledit orifice d'insertion (22) étant pourvu d'un clapet anti-retour (28),
dans lequel ledit clapet anti-retour (28) est configuré pour permettre à un dispositif de traitement intravasculaire (36) de passer à travers jusqu'à ladite extrémité proximale (14) et empêcher aussi du fluide corporel de s'écouler hors du vaisseau sanguin artificiel (10), dans lequel ledit clapet anti-retour (28) comporte un premier composant de clapet (32) qui empêche le fluide corporel de s'écouler au-dehors quand le dispositif de traitement intravasculaire (36) est inséré à travers l'orifice d'insertion (22) et un second composant de clapet (34) qui empêche le fluide corporel de s'écouler au-dehors quand le dispositif de traitement intravasculaire (36) n'est pas inséré à travers l'orifice d'insertion (22).

2. Vaisseau sanguin artificiel (10) selon la revendication 1, dans lequel l'extrémité distale (16a) forme un embranchement.

3. Vaisseau sanguin artificiel (10) selon la revendication 2, dans lequel ledit embranchement forme au moins une fourche à trois branches.

4. Vaisseau sanguin artificiel (10) selon la revendication 2 ou 3, dans lequel ledit orifice d'insertion (22) est positionné entre ledit embranchement et ladite extrémité proximale (14).

5. Vaisseau sanguin artificiel (10) selon la revendication 1, dans lequel
le second composant de clapet (34) est positionné plus proche du côté avant pour l'insertion du dispositif de traitement intravasculaire (36) que le premier composant de clapet (32),
le premier composant de clapet (32) comporte un trou (32a) qui permet au dispositif de traitement intravasculaire (36) d'être inséré de manière coulissante et vient en contact étroit avec la surface extérieure du dispositif de traitement intravasculaire (36) qui a été inséré, et
le second composant de clapet (34) comporte une paire de parois (34c) inclinées vers la direction dans laquelle le dispositif de traitement intravasculaire (36) est inséré dans l'orifice d'insertion (22), des extrémités adjacentes (34d) desdites parois (34c) inclinées sont configurées pour se rapprocher et se séparer l'une de l'autre de telle manière que le dispositif de traitement intravasculaire (36) peut passer à travers l'espace entre les parois (34c) inclinées.

6. Vaisseau sanguin artificiel (10) selon la revendication 1, dans lequel
l'extrémité proximale (14) peut être connectée à l'aorte ascendante (12c) et
l'extrémité distale (16a) peut être connectée à au moins un du tronc artériel brachiocéphalique droit (24a), de l'artère carotide commune gauche (24b), et de l'artère sous-clavière gauche (24c).
